# EUROPÄISCHE PATENTANMELDUNG

(11) **EP 3 443 926 A1**
(43) Veröffentlichungstag der Anmeldung: **20.02.2019**
(21) Anmeldenummer: 18187019.7
(22) Anmeldetag: 02.08.2018
(51) Int. Cl.: A61B 34/30, A61B 90/50, B25J 9/08, B25J 9/00

(54) **ROBOTISCHES OPERATIONSSYSTEM**

(30) Priorität: 09.08.2017 DE 102017118126
(71) Anmelder: avateramedical GmbH, 07745 Jena (DE)
(72) Erfinder: Hille, Leander, 76751 Jockgrim (DE); Luksch, Tobias, 76761 Rülzheim (DE); Tauro, Ricardo A., 76187 Karlsruhe (DE); Trommer, Christian, 99310 Wipfratal (DE)
(74) Vertreter: Patentanwälte Geyer, Fehners & Partner mbB

(57) **Zusammenfassung**

Die Erfindung betrifft ein robotisches Operationssystem, welches eine Tragsäule (2), eine Anzahl von Manipulatorarmen (9), eine Steuervorrichtung (19) zur Steuerung des robotischen Operationssystems und einen Ausleger (3) umfasst. Der Ausleger (3) ist an seinem einen Ende mit der Tragsäule (2) verbunden und weist an seinem anderen Ende Mittel zur Ankopplung der Manipulatorarme (9) mit einer der Anzahl von Manipulatorarmen (9) entsprechenden Anzahl von Koppelvorrichtung für die Manipulatorarme (9) auf. Erfindungsgemäß ist in Abhängigkeit von der Position des jeweiligen Manipulatorarms (9) am Ausleger (3) ein erster Teil der Anzahl von Manipulatorarmen (9) über erste Koppelvorrichtungen mit ersten Koppelmechanismen und ein zweiter Teil der Anzahl von Manipulatorarmen (9) über zweite Koppelvorrichtungen mit zweiten Koppelmechanismen mit dem Ausleger (3) verbunden. Der erste Koppelmechanismus besteht aus einem ersten Drehgelenk (5). Der zweite Koppelmechanismus besteht aus einem zweiten Drehgelenk (6) und einem dritten Drehgelenk (7) sowie einem die beiden Drehgelenke (6, 7) verbindenden Getriebeglied (8).

## Beschreibung

### Gebiet der Erfindung

Die Erfindung betrifft ein robotisches Operationssystem. Ein solches Operationssystem umfasst eine optional höhenverstellbare Tragsäule, eine Anzahl von Manipulatorarmen und einen Ausleger, der an seinem einen Ende mit der Tragsäule verbunden ist und an seinem anderen Ende Mittel zur Ankopplung der Manipulatorarme aufweist. Das robotische Operationssystem umfasst außerdem eine Steuervorrichtung zur Steuerung des robotischen Operationssystems, insbesondere zur Vorpositionierung der Manipulatorarme vor einer Operation.

Bei einem robotischen Operationssystem bedient der Operateur, der Chirurg, einen oder mehrere robotisch gesteuerte Arme, welche Instrumente halten, um einzelne Schritte einer Operation durchzuführen. Der Chirurg steuert die Manipulatorarme des Systems über ein Bedienpult oder durch entsprechende Bewegungen über einen geeigneten Koppelmechanismus, der Bewegungen der Hände des Chirurgen in Bewegungen des entsprechenden Instruments umsetzt. Die Manipulatorarme sind vielgliedrige Systeme, bei denen die einzelnen Glieder über Gelenke miteinander verbunden sind. Ein Teil der Glieder dient der Positionierung des Manipulatorarms relativ zu anderen Manipulatorarmen desselben robotischen Systems, so dass die Manipulatorarme sich nicht gegenseitig behindern. Dieser Teil der Glieder wird auch als Stellvorrichtung bezeichnet. Die beschriebene Positionierung erfolgt vor der Operation und wird daher auch als Vorpositionierung bezeichnet. Ein anderer Teil der Glieder dient der Positionierung und Führung des Werkzeugs am Objekt, also beispielsweise der Positionierung eines Operationsinstruments oder eines Endoskops im Bereich einer Öffnung in der Gewebedecke eines Patienten. Dieser Teil der Glieder wird auch als Instrumententrägervorrichtung bezeichnet. Die Armglieder sind im kinematischen Sinne als offene kinematische Kette miteinander verbunden, da am letzten Glied kein weiteres Gelenk sitzt, sondern das Werkzeug. Alle anderen Glieder sind nach Art einer Kette über Gelenke miteinander verbunden.

An dem Ende der Kette, welches nicht das Instrument hält, ist die Kette mit einer Trägervorrichtung - umfassend auch die Tragsäule und den Ausleger - über eine Kopplungsvorrichtung verbunden. Die Trägervorrichtung dient insbesondere der Halterung der einzelnen Manipulatorarme, in ihr werden aber auch die elektrischen Leitungen geführt, welche der Stromversorgung und der Steuerung der Manipulatorarme dienen. Die Instrumententrägervorrichtungen selbst mit den Instrumenten operieren in einem steril zu haltenden Bereich, an ihnen dürfen keine Leitungen offen liegen.

Die Manipulatorarme werden einzeln angesteuert, sind jedoch an die gemeinsame Trägervorrichtung angekoppelt, so dass ein Chirurg oder medizinisches Hilfspersonal auch weiterhin einen einfachen Zugang zum behandelten Patienten hat. Aufgrund der Masse der Arme muss die Trägervorrichtung, wenn sie auf dem Boden gelagert wird, entsprechend groß dimensioniert werden, um ein ausreichendes Gegengewicht zu den Manipulatorarmen zu erzeugen. Andererseits soll das Operationssystem auch so kompakt wie möglich gehalten werden, um eine gute Zugänglichkeit zum Patienten und eine einfache Handhabbarkeit des Operationssystems, wenn dieses beispielsweise bewegt werden muss, zu gewährleisten.

Um eine maximale Beweglichkeit der Manipulatorarme des Operationssystems zu ermöglichen, sollten diese idealerweise unabhängig voneinander aufgehängt werden, was in der Praxis jedoch zu aufwendig ist und andere Nachteile mit sich bringt. Beispielsweise könnten vier Manipulatorarme an festen Positionen an der Patientenliege angeordnet sein. Dies erschwert allerdings den Zugang zum Patienten durch Personal. In der Praxis wird daher immer eine gemeinsame Aufhängung der - meistens vier - Manipulatorarme verwendet, was jedoch unter Umständen dazu führen kann, dass sich die Manipulatorarme gegenseitig behindern, bzw. nicht so frei einstellbar sind, wie es für eine optimale Durchführung der Behandlung wünschenswert wäre.

### Stand der Technik

Im Stand der Technik sind für die Aufhängung der Manipulatorarme verschiedene Vorrichtungen bekannt, die Mittel zur Ankopplung der Manipulatorarme also auf verschiedene Weise realisiert.

Bei dem in der DE 10 2013 004 459 A1 offenbarten Operationssystem mit vier Manipulatorarmen sind die Manipulatorarme über identische Kopplungsvorrichtungen mit einem Ausleger mit einer T-förmigen oder querträgerartigen Struktur verbunden und können um die Längsachse des Querträgers geschwenkt werden. Der Querträger selbst weist Gelenke auf, die eine Verkippung gegen die Längsachse des Querträgers um etwa 30° zulässt. Dies führt jedoch während des Operationsbetriebs unter Umständen zu Kollisionen zwischen den Armen. Die das geschlossene Ende der kinematischen Kette bildenden Manipulatorarmglieder sind im Bereich der Ankopplungspunkte an die Trägervorrichtung in Bezug auf die Längsachse des Querträgers entlang dieser immer gleich beabstandet.

In der US 6,837,883 B2 wird ein robotisches Operationssystem beschrieben, bei dem vier Manipulatorarme einzeln an den vier Seiten einer quaderförmigen Säule angebracht sind, die Arme sind einzeln höhenverstellbar. Die vier Arme werden hier also nicht an einem gemeinsamen Ausleger befestigt, sondern individuell an der Tragsäule, an der die Arme einzeln höhenverstellbar sind. Die Tragsäule insgesamt muss daher nicht höhenverstellbar sein. Der hintere Arm weist dabei ein weiteres Armsegment auf, um den größeren Abstand zum Patienten zu kompensieren. Um die Funktionalität der Vorpositionierung erfüllen zu können, müssen die Armsegmente vergleichsweise lang sein, die Vorrichtung ist daher insgesamt sehr raumgreifend, was ihre Handhabbarkeit erschwert.

Ein weiteres robotisches Operationssystem wird in der US 2014/0052153 A1 beschrieben. Die Tragsäule ist hier höhenverstellbar, an der Tragsäule ist ein horizontal abstehender Ausleger angebracht, dieser ist mit seiner einer Seite mit der Tragsäule fest verbunden, wie beispielsweise in Fig. 8 der US 2014/0052153 A1 gezeigt. An der freien Seite des Auslegers ist eine sogenannte Orientierungsplattform angeordnet. An der Orientierungsplattform sind in horizontaler Richtung - genauer in der Ebene der Plattform - abstehend vier Armausleger angeordnet, die individuell in der Ebene geschwenkt sowie in ihrer Länge angepasst werden können. Zusätzlich ist die Orientierungsplattform selbst auch um eine vertikale Rotationsachse drehbar. An den freien Enden der Armausleger sind schließlich die Manipulatorarme des robotischen Operationssystems befestigt, wobei das erste Armglied, welches mit dem jeweiligen Armausleger verbunden wird, mit seiner Längsachse in vertikaler Richtung orientiert ist und um diese rotierbar mit dem Armausleger verbunden ist. Die Orientierungsplattform ist außerdem bezüglich der horizontalen Basisebene verkippbar, was zu einer Lagenänderung der eben beschriebenen Rotationsachsen, Armausleger, Orientierungen und Armrichtungen führt. Dieser Aufbau bietet zwar viele Einstellungsmöglichkeiten, ist jedoch sehr komplex aufgebaut, was zum einen eine technisch und die Kosten betreffend aufwendige Herstellung erfordert sowie aufgrund der vielen Einstellungsmöglichkeiten eine lange Eingewöhnungszeit des Bedieners.

### Beschreibung der Erfindung

Aufgabe der Erfindung ist es daher, ein robotisches Operationssystem der eingangs beschriebenen Art dahingehend weiterzuentwickeln, dass mit einer möglichst einfach konstruierten Ankopplungsvorrichtung für die Manipulatorarme dennoch eine ausreichend große Bewegungsfreiheit für die Arme ermöglicht wird, die den Chirurg nicht behindert, bzw. Kollisionen während des Betriebs vermeidet. Dabei soll das System außerdem möglichst kompakt gebaut sein, so dass es im Einsatz wenig Platz beansprucht.

Diese Aufgabe wird für ein robotisches Operationssystem der eingangs beschriebenen Art dadurch gelöst, dass in Abhängigkeit von der Position des jeweiligen Manipulatorarms am Ausleger ein erster Teil der Anzahl von Manipulatorarmen über erste Koppelvorrichtungen mit ersten Koppelmechanismen und ein zweiter Teil der Anzahl von Manipulatorarmen über zweite Koppelvorrichtungen mit zweiten Koppelmechanismen mit dem Ausleger verbunden ist. Verschiedene Manipulatorarme können also mit verschiedenen Koppelvorrichtungen an den Ausleger angekoppelt werden. Die Auswahl der jeweils zu verwendenden Koppelvorrichtung erfolgt dabei in Abhängigkeit von der Position des jeweiligen Manipulatorarms am Ausleger. Indem nicht für alle Manipulatorarme die gleiche Koppelvorrichtung verwendet wird, wie dies im Stand der Technik der Fall ist, erhält man eine größere Flexibilität. Da jedoch die Koppelvorrichtungen auf einen relativ kleinen Bauraum beschränkt sind, da sie alle am Ausleger angebracht sind, geht dieser Zugewinn an Flexibilität nicht zu Lasten der Kompaktheit.

Die Koppelvorrichtung ist dabei zwischen den Ausleger und den Manipulatorarm zwischengeschaltet, verbindet diese beiden also. Der erste Koppelmechanismus in der ersten Koppelvorrichtung besteht dabei aus einem ersten Drehgelenk. In einer kompakten Ausfertigung befindet sich zwischen dem Ausleger und dem daran über die erste Koppelvorrichtung angekoppelten Manipulatorarm eine Gleitebene senkrecht zur Rotationsachse des ersten Drehgelenks, der Abstand kann jedoch auch größer sein, so dass die Bauform des Auslegers die Bewegung des Manipulatorarms möglichst wenig behindert. Der zweite Koppelmechanismus besteht aus einem zweiten Drehgelenk, einem dritten Drehgelenk und einem Getriebeglied, welche das zweite und das dritte Drehgelenk miteinander verbindet. Die Rotationsachsen des zweiten Drehgelenks und des dritten Drehgelenks liegen parallel zueinander, mittels des Getriebegliedes wird außerdem einerseits ein Abstand zwischen den beiden Rotationsachsen senkrecht zu diesen hergestellt, d.h. mittels des Getriebegliedes sind die Rotationsachsen des zweiten und des dritten Drehgelenks und damit die beiden Drehgelenke in einer Richtung senkrecht zu den Rotationsachsen voneinander beabstandet. Andererseits gelingt es mittels des Getriebegliedes auch, einen Abstand der Drehgelenke in Richtung entlang der Rotationsachsen herzustellen, d.h. mittels des Getriebegliedes sind die beiden Drehgelenke auch in Richtung entlang der Rotationsachsen voneinander beabstandet.

Das zweite Drehgelenk verbindet den Ausleger mit dem Getriebeglied und das dritte Drehgelenk das Getriebeglied mit dem Manipulatorarm. Durch das Getriebeglied wird ein Versatz der beiden Drehgelenke zueinander entlang einer Richtung parallel zu den Rotationsachsen hergestellt, so dass die Rotationen um das zweite und das dritte Drehgelenk unabhängig voneinander und insbesondere vom Grundsatz her unbeeinflusst voneinander erfolgen können. Bei zwei als Scharnieren ausgestalteten Drehgelenken befindet sich das Getriebeglied genau dazwischen, so dass sich die Rotationen um die beiden Gelenke vom Prinzip her nicht gegenseitig behindern können. In der Praxis wird die Bewegungsfreiheit jedoch durch die Abmessungen des Auslegers und durch die anderen Manipulatorarme eingeschränkt. Wesentlich ist jedoch der vertikale Versatz des zweiten und des dritten Drehgelenks zueinander, der durch das Getriebeglied hergestellt wird, so dass der am Getriebeglied angebrachte Manipulatorarm eine möglichst große Bewegungsfreiheit erhält.

Das Getriebeglied des zweiten Koppelmechanismus kann beispielsweise als stabförmiger oder plattenförmiger Körper ausgestaltet sein. Vorteilhaft weist es zwei einander gegenüberliegende Seiten auf, die entlang der Rotationsachsen des zweiten bzw. dritten Drehgelenks beabstandet sind; diese Seiten werden ohne Beschränkung der Allgemeinheit auch als Ober- und Unterseite bezeichnet. Die Drehachsen des zweiten und des dritten Drehgelenks verlaufen beide auch durch das Getriebeglied und durchstoßen jeweils Ober- und Unterseite. Zweckmäßig sollte dabei der Abstand zwischen den beiden Rotationsachsen so groß wie möglich gewählt werden, um eine maximale Bewegungs- und Einstellmöglichkeit zu erhalten. Das zweite Drehgelenk verbindet das Getriebeglied an der einen der beiden gegenüberliegenden Seiten mit dem Ausleger. Das dritte Drehgelenk verbindet das Getriebeglied an der anderen der beiden einander gegenüberliegenden Seiten mit dem Manipulatorarm.

Welcher Manipulatorarm mit welcher Kopplungsvorrichtung an dem Ausleger angekoppelt wird, hängt von der Position der Koppelvorrichtung am Ausleger ab. Sind beispielsweise die Koppelvorrichtungen am Ausleger nach Art einer Reihe angeordnet, beispielsweise reihenförmig an einem optionalen Querträger, so ist es vorteilhaft, wenn mindestens an den beiden Endpositionen der Reihe die Manipulatorarme mittels der zweiten Koppelvorrichtungen mit dem Ausleger verbunden sind. Auf diese Weise erhalten insbesondere die außenliegenden Arme, die etwas weiter weg vom Behandlungsort liegen, eine größere Bewegungsfreiheit.

Bevorzugt ist der Ausleger an seinem der Tragsäule abgewandten Ende jedoch nicht reihenförmig, beispielsweise mit einem Querträger, ausgebildet, sondern als zweizinkige, Y-förmige Gabelstruktur, wobei an den Zinken, besonders bevorzugt an deren freien oder losen Enden, jeweils ein Manipulatorarm mittels einer zweiten Koppelvorrichtung mit dem Ausleger verbunden ist. Dadurch werden die äußeren Manipulatorarme noch etwas näher am Behandlungsort positioniert und müssen nicht um die innere Arme herumgreifen. So wird eine noch kompaktere Bauweise ermöglicht.

Durch die Drehung der Manipulatorarme um die Rotationsachsen der ersten, zweiten und dritten Drehgelenke soll eine Vorpositionierung der Manipulationsarme für die Operation erreicht werden, so dass im Anschluss nur noch die Instrumententrägervorrichtung während der Operation bewegt wird. Zu diesem Zweck ist es vorteilhaft, wenn die Drehgelenke motorisch angetrieben und allesamt motorisch einstellbar und fixierbar sind, insbesondere dass beim zweiten Koppelmechanismus die Relativposition des dritten Drehgelenks in Bezug auf das zweite Drehgelenk motorisch einstellbar und / oder über eine bevorzugt motorisch stellbare Bremse fixierbar ist.

Die Tragsäule kann, wie es im Stand der Technik üblich ist, bodengestützt sein, in einer besonders bevorzugten Ausgestaltung ist sie jedoch deckengestützt, d.h. sie hängt von der Decke herab. Dies verbessert die Zugangsmöglichkeiten für das medizinische Personal zum Patienten erheblich.

Der Ausleger kann mit der Tragsäule einen rechten Winkel einschließen, er kann aber auch mindestens teilweise bogenförmig ausgebildet sein, der vom Bogen eingeschlossene Winkel beträgt dann vorzugsweise ebenfalls 90°. Auch die Bogenform trägt zu einer kompakteren Bauweise bei. Insbesondere wenn der Ausleger von der Tragsäule abstehend ausgebildet ist, kann es vorteilhaft sein, wenn der Ausleger auch in seiner Länge verstellbar ist, um die Manipulatorarme noch näher an Patienten positionieren zu können, ohne dass die Tragsäulenstruktur stört.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

### Kurze Beschreibung der Zeichnungen

Nachfolgend wird die Erfindung beispielsweise anhand der beigefügten Zeichnungen, die auch erfindungswesentliche Merkmale offenbaren, noch näher erläutert. Es zeigen:
- Fig. 1a): den beispielhaften Aufbau einer Tragestruktur für ein robotisches Operationssystem,
- Fig. 1b): die Gliederstruktur dieses Trägeraufbaus,
- Fig. 1c): einen zweiten Koppelmechanismus,
- Fig. 1d): eine abgewandelte Gliederstruktur für einen Trägeraufbau,
- Fig. 2: eine Perspektivansicht eines Ausschnitts einer Trägerstruktur für ein robotisches Operationssystem,
- Fig. 3: eine Draufsicht auf das in Fig. 2 gezeigte System,
- Fig. 4: an die Trägerstruktur angekoppelte Teile von Manipulatorarmen ohne Instrumenten-trägervorrichtung,
- Fig. 5: einen beispielhaften Manipulatorarm mit Instrumententrägervorrichtung,
- Fig. 6 a): eine Steuervorrichtung eines robotischen Operationssystems und
- Fig. 6b): ein robotisches Operationssystem mit Patientenliege.

### Ausführliche Beschreibung der Zeichnungen

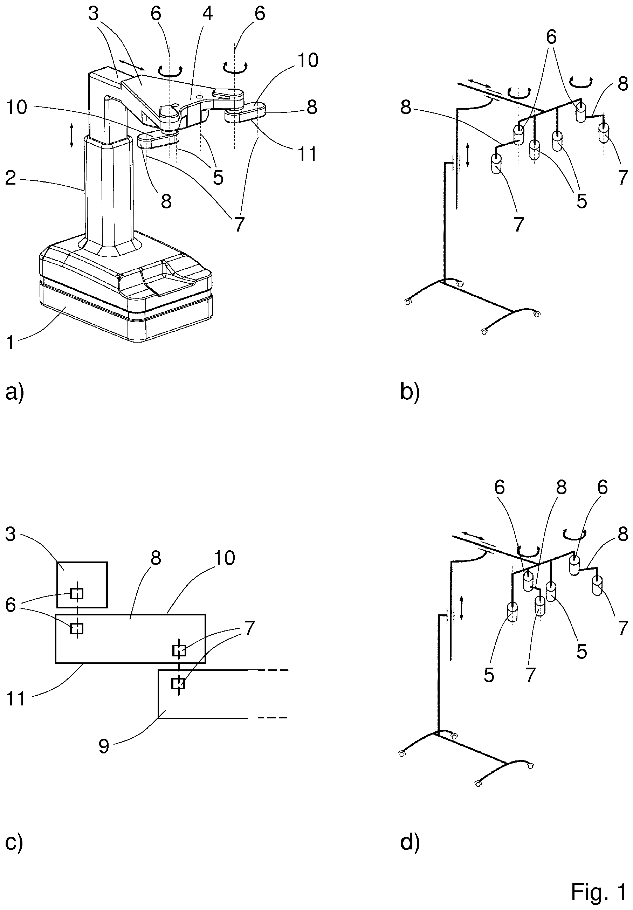

In Fig. 1a) ist eine beispielhafte Trägerstruktur für ein robotisches Operationssystem gezeigt. Diese umfasst einen Standfuß 1, der im Inneren durch Gewichte beschwert sein kann und / oder auch mit dem Boden verschraubt oder anderweitig verbunden sein kann. Aus dem Standfuß 1 erhebt sich eine Tragsäule 2, die hier vorteilhaft in ihrer Höhe verstellbar ist. Am oberen Ende der Tragsäule 2 ist ein Ausleger 3 von der Tragsäule in einem Winkel von 90° abstehend ausgebildet. Andere Winkel sind selbstverständlich ebenfalls möglich. Der Ausleger 3 ist hier gerade ausgeführt und in seiner Länge verstellbar. An seinem der Tragsäule 2 abgewandten, freien Ende ist der Ausleger 3 als zweizinkige, Y-förmige Gabelstruktur 4 ausgebildet. Der Ausleger 3 weist an diesem Ende Mittel zur Ankopplung von Manipulatorarmen mit einer der Anzahl von Manipulatorarmen entsprechenden Anzahl von Koppelvorrichtungen für die Manipulatorarme auf. Nicht gezeigt ist eine Steuervorrichtung zur Steuerung des robotischen Operationssystems, die jedoch einen Bestandteil des Systems bildet. Alternativ zur Ausbildung als Gabelstruktur 4 kann der Ausleger 3 an seinem freien Ende auch eine querträgerartige Struktur, eine Traverse aufweisen, an der die Koppelvorrichtungen aufgereiht sind.

In Abhängigkeit von der Position des jeweiligen Manipulatorarms am Ausleger 3 ist ein erster Teil der Anzahl von Manipulatorarmen über erste Koppelvorrichtungen mit ersten Koppelmechanismen mit dem Ausleger 3 verbunden. Ein zweiter Teil der Anzahl von Manipulatorarmen ist über zweite Koppelvorrichtungen mit zweiten Koppelmechanismen mit dem Ausleger 3 verbunden. Der erste Koppelmechanismus besteht aus einem ersten Drehgelenk 5, hier entsprechend durch gestrichelte Linien angedeutet. Der zweite Koppelmechanismus besteht aus einem zweiten Drehgelenk 6 und einem dritten Drehgelenk 7 sowie einem die beiden Drehgelenke 6, 7 verbindenden Getriebeglied 8. Alle Koppelvorrichtungen können motorische Stellantriebe für das erste, zweite und/oder dritte Drehgelenk 5, 6, 7 umfassen. Die Rotationsachse des zweiten und des dritten Drehgelenks 6, 7 liegen zueinander parallel und sind mittels des Getriebeglieds 8 zueinander in einer Richtung senkrecht zu den Rotationsachsen beabstandet. Das Getriebeglied 8 sorgt außerdem dafür, dass das zweite Drehgelenk 6 und dass dritte Drehgelenk 7 auch in Richtung der Rotationsachsen, d.h. entlang dieser, voneinander beabstandet sind, so dass sich Rotationsbewegungen um das zweite und das dritte Drehgelenk 6, 7 im Prinzip nicht behindern.

Dies ist abstrahiert und zur Verdeutlichung noch einmal in Fig. 1b) gezeigt, hier sind die Aufbauten weggelassen und nur die Gliederstruktur für die Bewegungen, die den Koppelvorrichtungen aufgeprägt werden können, sind dargestellt. Zum einfacheren Verständnis wurden die gleichen Bezugszeichen wie in Fig. 1a) gewählt, sie sind hier jedoch abstrahiert zu verstehen. Die ersten Drehgelenke 5 sind hier auf der gleichen Höhe wie die zweiten Drehgelenke 6 eingezeichnet, während sie bei der in Fig. 1a) gezeigten Variante auf der gleichen Höhe wie die dritten Drehgelenke 7 angeordnet sind, so dass alle Manipulatorarme auf der gleichen Höhe ankoppeln.

Fig. 1c) erläutert den zweiten Koppelmechanismus etwas näher. Über das zweite Drehgelenk 6 ist der Ausleger 3 mit dem Getriebeglied 8 verbunden. Über das dritte Drehgelenk 7 ist das Getriebeglied 8 seinerseits mit einem Manipulatorarm 9 verbunden. Das Getriebeglied 8 sorgt einerseits dafür, dass die Rotationsachsen des zweiten Drehgelenks 6 und des dritten Drehgelenks 7 in einer Richtung senkrecht zu den Rotationsachsen voneinander beabstandet sind. Andererseits sorgt das Getriebeglied 8 auch dafür, dass ein Versatz zwischen dem zweiten Drehgelenk 6 und dem dritten Drehgelenk 7 entlang der Rotationsachsen eingeführt wird, der dafür sorgt, dass sich die Rotationsbewegungen der beiden Gelenke grundsätzlich nicht behindern, d.h. die Rotation des Manipulatorarms 9 um die Rotationsachse des dritten Drehgelenks 7 nicht durch die Rotation des Getriebegliedes 8 um die Rotationsachse des zweiten Drehgelenks 6 behindert wird, sofern nicht eine Behinderung durch die Abmessungen des Auslegers 3 etc. erfolgt.

Das Getriebeglied 8 ist in Fig. 1c) als dreidimensionaler Körper mit zwei einander gegenüberliegenden Seiten, hier einer Oberseite 10 und einer Unterseite 11 ausgebildet. Grundsätzlich kann das Getriebeglied 8 auch als flacher plattenförmiger Körper ausgebildet sein, sofern dieses den Belastungen durch die Masse der daran anzubringenden Manipulatorarme 9 standzuhalten in der Lage ist. Der Ausleger 3 ist an der Oberseite 10 des Getriebegliedes 8 mit diesem über das zweite Drehgelenk 6 verbunden, der Manipulatorarm ist an der Unterseite 11 des Getriebegliedes 8 mit diesem über das dritte Drehgelenk 7 verbunden. In dem hier gezeigten kompakten Aufbau befindet sich zwischen dem Ausleger 3 und dem Getriebeglied 8 und zwischen dem Getriebeglied 8 und dem Manipulatorarm 9 jeweils ein Luftabstand. In einer noch kompakteren Ausführung können die Luftabstände auch als erste und zweite Gleitebene ausgebildet sein. Beide Gleitebenen sind durch die Abmessungen des Getriebegliedes 8 entlang der Rotationsachsen der Drehgelenke 6, 7 voneinander beabstandet. Das Getriebeglied kann auch stabförmig mit rundem Querschnitt ausgebildet sein. Die Oberseite und die Unterseite entsprechend dann den jeweiligen oberen und unteren kreisbogenförmigen Flächen mit jeweils einem Winkel im Querschnitt von 180°. Beliebige Zwischenformen und andere Formen sind ebenfalls möglich, die beiden gegenüberliegenden Seiten können auch als nahezu eindimensionale Linienstrukturen ausgebildet sein, beispielsweise bei einem plattenförmigen Körper, dessen Großflächen eine Normalenrichtung senkrecht zu den Rotationsachsen aufweisen.

Fig. 1d) zeigt eine gegenüber Fig. 1a) und Fig. 1b) leicht abgewandelte Konfiguration. Während die in Fig. 1a), 1b) gezeigte Konfiguration symmetrisch in der Weise ist, dass die - in diesem Fall zwei - Manipulatorarme 9, die den zweiten Teil der Anzahl von Manipulatorarmen 9 bilden, außen angeordnet sind, und die übrigen Manipulatorarme 9 innen, so entspricht die in Fig. 1d) gezeigte Konfiguration einer alternierenden Anordnung, bei der ein Manipulatorarm 9 der ersten Anzahl von Manipulatorarmen 9 ausschließlich neben Manipulatorarmen 9 der zweiten Anzahl von Manipulatorarmen 9 angeordnet ist. Daneben sind auch weitere Anordnungsmöglichkeiten der ersten und zweiten Anzahl von Manipulatorarmen 9 möglich, beispielsweise eine zu Fig. 1a), b) komplementäre Anordnung, d.h. eine Anordnung, bei der die erste Anzahl von Manipulatorarmen 9 außen und die zweite Anzahl von Manipulatorarmen innen angeordnet ist. Je mehr Manipulatorarme 9 das robotische Operationssystem umfasst, desto mehr Möglichkeiten der Anordnung der ersten und der zweiten Anzahl von Manipulatorarmen 9 gibt es, bis hin zu einer statistischen oder als Ergebnis eines Rechenprozesses als besonders vorteilhaft bestimmten Anordnung mit minimaler gegenseitiger Behinderung der Bewegungen der Manipulatorarme 9.

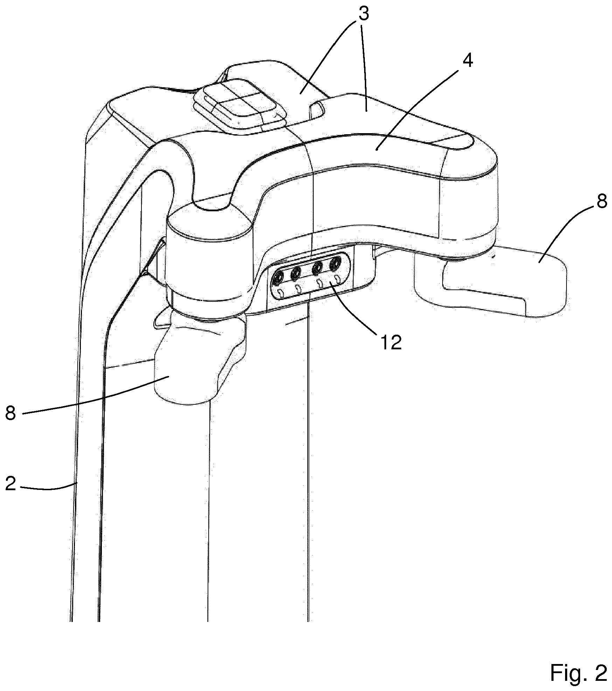

Fig. 2 zeigt eine ähnliche Ausgestaltung wie Fig. 1a), hier ist jedoch der Ausleger 3 nicht in seiner Länge verstellbar. Fig. 2 zeigt einen Ausschnitt eines Trägersystems, der Standfuß ist hier nicht dargestellt. Hier sind außerdem Ausgänge eines Bussystems 12 gezeigt, welche zur Einkopplung der Steuerung der Manipulatorarme dient. Die Relativposition des dritten Drehgelenks 7 in Bezug auf das zweite Drehgelenk 6 und die Positionen aller Manipulatorarme 9 lassen sich mittels der Steuerung motorisch einstellen, ergänzend sind die Relativpositionen der dritten Drehgelenke 7 über eine motorisch stellbare Bremse fixierbar.

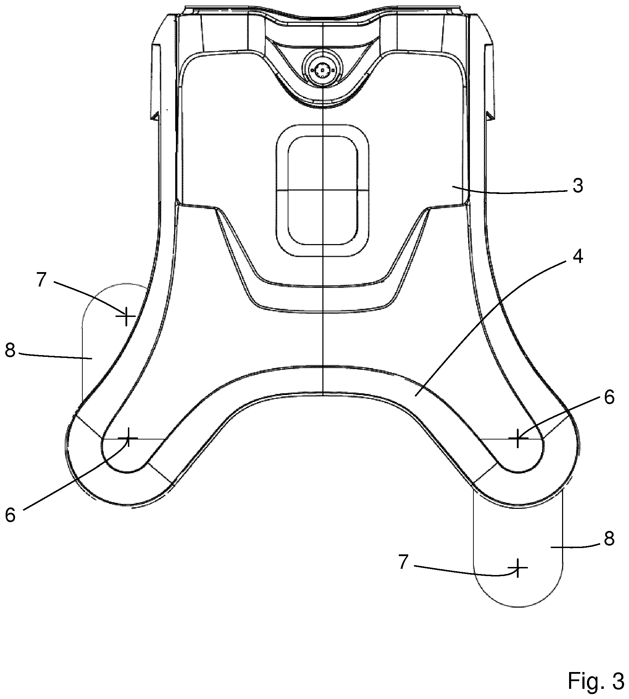

Fig. 3 zeigt das Trägersystem aus Fig. 2 in einer Draufsicht von oben, hier sind die Getriebeglieder 8 in zwei extremen Positionen gezeigt, um die Bewegungsmöglichkeiten zu verdeutlichen. Die Positionen der zweiten Drehgelenke 6 und die Momentanpositionen der dritten Drehgelenke 7 sind durch Kreuze gekennzeichnet.

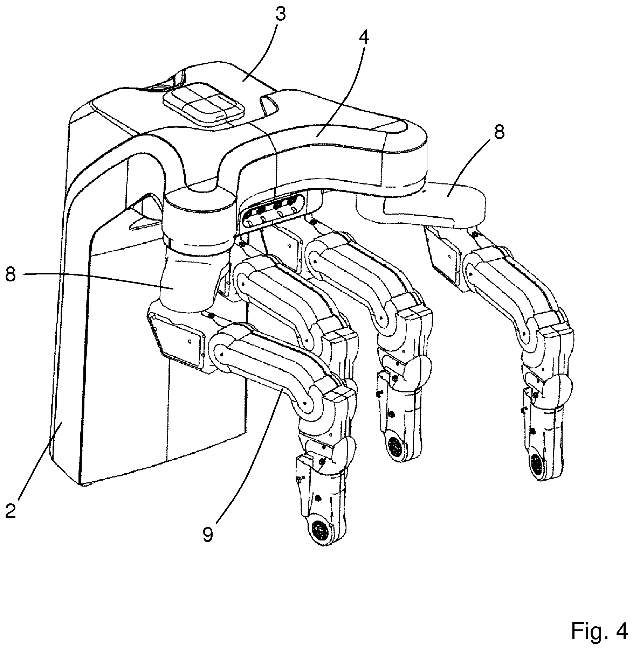

Fig. 4 zeigt schließlich einen Ausschnitt des robotischen Operationssystems, dessen Trägerstruktur in den Fig. 2 und 3 dargestellt war, hier mit Teilen angekoppelter Manipulatorarme 9. Die hier gezeigten Teile der Manipulatorarme 9 dienen der Vorpositionierung, an die Teile ist noch eine Instrumententrägervorrichtung anzukoppeln.

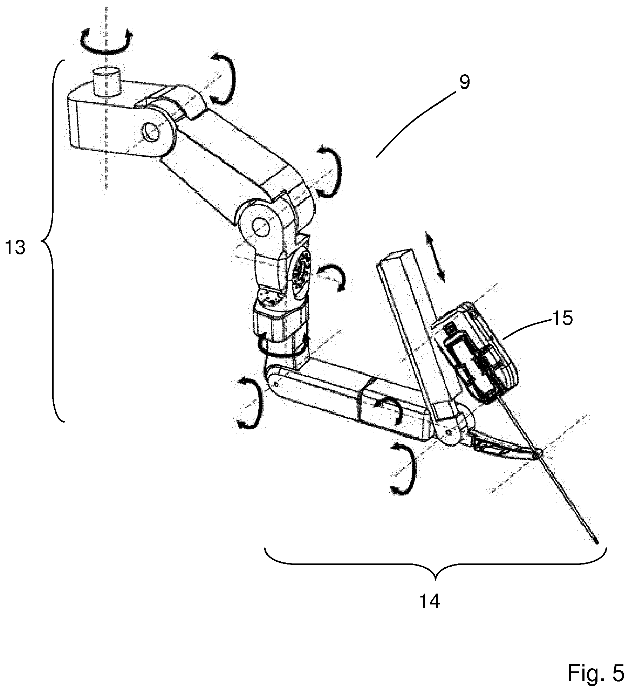

Der Vollständigkeit halber ist in Fig. 5 ein vollständiger Manipulatorarm 9 gezeigt. Bei dem Arm handelt es sich um ein vielgliedriges System, was einer losen kinematischen Kette entspricht. Ein Teil der Glieder bildet eine Positionierungsvorrichtung 13, zusammengefasst durch die geschweifte Klammer linkt in der Zeichnung, der andere Teil der Glieder bildet die Instrumententrägervorrichtung 14 mit daran angekoppeltem Instrumentenhalter 15.

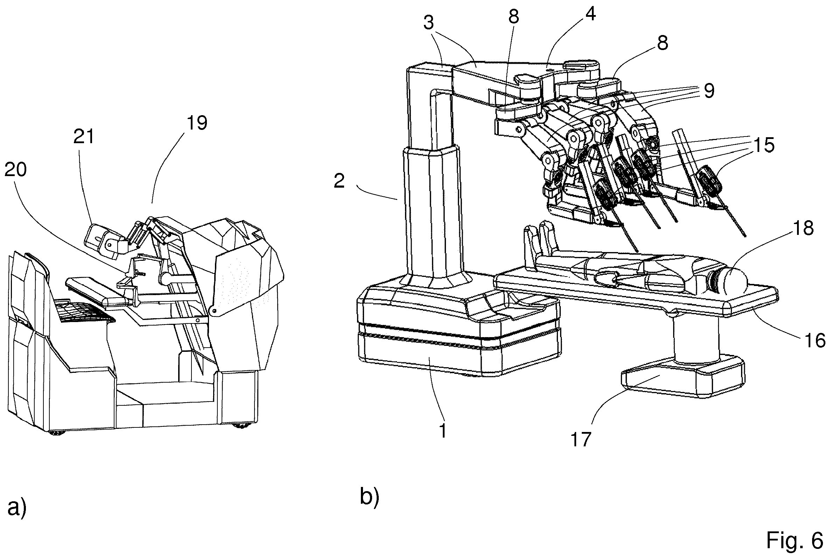

Fig. 6a, b) schließlich zeigt ein robotisches Operationssystem in einer Gesamtansicht. In Fig. 6b) ist die Trägerstruktur des robotischen Operationssystems gezeigt, die analog zu Fig. 1a) einen Standfuß 1, eine Tragsäule 2 mit einem davon abstehenden Ausleger 3 und einer Gabelstruktur 4 am freien Ende des Auslegers 3 umfasst. Am Ausleger 3 mit der Gabelstruktur 4 sind insgesamt vier Manipulatorarme 9 mit Instrumentenhaltern 15 angeordnet, die äußeren beiden bilden wieder die zweite Anzahl von Manipulatorarmen 9. Diese befinden sich hier in einer Ruhestellung und sind noch nicht für eine Operation positioniert. Unterhalb der Manipulatorarme 9 mit den Instrumentenhaltern 15 ist eine Patientenliege 16 auf einem Liegenstandfuß 17 angeordnet, auf der sich eine zu operierende Person 18 befindet. Die Patientenliege 16 kann auf dem Liegenstandfuß 17 drehbar gelagert sein. Darüber hinaus können die Trägerstruktur des robotischen Operationssystems und die Patientenliege 16 relativ zueinander vorteilhaft auch translatorisch bewegt werden.

Das robotische Operationssystem verfügt auch über eine Steuervorrichtung 19 zu seiner Steuerung, welche in Fig. 6a) gezeigt ist und gegenüber der Trägerstruktur ebenfalls relativ bewegbar, hier verfahrbar, ist. Sie wird von einem speziell ausgebildeten Chirurgen bedient, und die Bewegungen des Chirurgen an Handgriffen 20 der Steuervorrichtung 19 werden auf die Manipulatorarme 9 bzw. auf in den Instrumentenhalterungen 15 gehaltene Instrumente übertragen. Der Chirurg kann dabei mit Hilfe einer Betrachtungseinrichtung 21 das Operationsfeld überwachen.

Das vorangehend beschriebene robotische Operationssystem kann kompakt gebaut werden und beansprucht aufgrund seiner Ankoppelstruktur für die Manipulatorarme im Vergleich zu herkömmlichen Operationssystemen weniger Platz, ermöglicht jedoch einem Chirurgen nichtsdestoweniger die Manipulationsarme kollisionsfrei zu positionieren.

### Bezugszeichenliste

- 1: Standfuß
- 2: Tragsäule
- 3: Ausleger
- 4: Gabelstruktur
- 5: erstes Drehgelenk
- 6: zweites Drehgelenk
- 7: drittes Drehgelenk
- 8: Getriebeglied
- 9: Manipulatorarm
- 10: Oberseite
- 11: Unterseite
- 12: Bussystem
- 13: Positioniervorrichtung
- 14: Instrumententrägervorrichtung
- 15: Instrumentenhalter
- 16: Patientenliege
- 17: Liegenstandfuß
- 18: Person
- 19: Steuervorrichtung
- 20: Handgriffe
- 21: Betrachtungseinrichtung

## Patentansprüche

1. Robotisches Operationssystem, umfassend
- eine Tragsäule (2),
- eine Anzahl von Manipulatorarmen (9),
- einen Ausleger (3), der an seinem einen Ende mit der Tragsäule (2) verbunden ist und an seinem anderen Ende Mittel zur Ankopplung der Manipulatorarme (9) mit einer der Anzahl der Manipulatorarme (9) entsprechenden Anzahl von Koppelvorrichtungen für die Manipulatorarme (9) aufweist, und
- eine Steuervorrichtung (19) zur Steuerung des robotischen Operationssystems, **dadurch gekennzeichnet, dass**
- in Abhängigkeit von der Position des jeweiligen Manipulatorarms (9) am Ausleger (3) ein erster Teil der Anzahl von Manipulatorarmen (9) über erste Koppelvorrichtungen mit ersten Koppelmechanismen und ein zweiter Teil der Anzahl von Manipulatorarmen (9) über zweite Koppelvorrichtungen mit zweiten Koppelmechanismen mit dem Ausleger (3) verbunden ist,
- wobei der erste Koppelmechanismus aus einem ersten Drehgelenk (5) besteht, und
- wobei der zweite Koppelmechanismus aus einem zweiten Drehgelenk (6), einem dritten Drehgelenk (7) und einem das zweite Drehgelenk (6) und das dritte Drehgelenk (7) verbindenden Getriebeglied (8) besteht, wobei das zweite Drehgelenk (6) und das dritte Drehgelenk (7) parallel zueinander liegende Rotationsachsen aufweisen und mittels des Getriebeglieds (8) sowohl in Richtung entlang der Rotationsachsen als auch in einer Richtung senkrecht zu den Rotationsachsen voneinander beabstandet sind.

2. Robotisches Operationssystem nach Anspruch 1, **dadurch gekennzeichnet, dass** das Getriebeglied (8) des zweiten Koppelmechanismus zwei einander gegenüberliegende Seiten aufweist, welche entlang der Rotationsachsen des zweiten Drehgelenks (6) und des dritten Drehgelenks (7) voneinander beabstandet sind, wobei das zweite Drehgelenk (6) an der einen der beiden gegenüberliegenden Seiten das Getriebeglied (8) mit dem Aufleger (3) verbindet und das dritte Drehgelenk (7) an der anderen der beiden gegenüberliegenden Seiten das Getriebeglied (8) mit einem Manipulatorarm (9) verbindet.

3. Robotisches Operationssystem nach einem der vorgenannten Ansprüche, **dadurch gekennzeichnet, dass** die Koppelvorrichtungen am Ausleger (3) reihenförmig angeordnet sind und mindestens an den beiden Endpositionen der Reihe die Manipulatorarme (9) mittels der zweiten Koppelvorrichtungen mit dem Ausleger (3) verbunden sind.

4. Robotisches Operationssystem nach einem der vorgenannten Ansprüche, **dadurch gekennzeichnet, dass** der Ausleger (3) an seinem der Tragsäule (2) abgewandten Ende als zweizinkige, Y-förmige Gabelstruktur (4) ausgebildet ist und an den Zinken, bevorzugt an deren losen Enden, jeweils ein Manipulatorarm (9) mit einer zweiten Koppelvorrichtung mit dem Ausleger verbunden ist.

5. Robotisches Operationssystem nach einem der vorgenannten Ansprüche, **dadurch gekennzeichnet, dass** beim zweiten Koppelmechanismus die Relativposition des dritten Drehgelenks (7) in Bezug auf das zweite Drehgelenk (6) motorisch einstellbar und / oder über eine bevorzugt motorisch stellbare Bremse fixierbar ist.

6. Robotisches Operationssystem nach einem der vorgenannten Ansprüche, **dadurch gekennzeichnet, dass** die Tragsäule (2) deckengestützt ist.

7. Robotisches Operationssystem nach einem der vorgenannten Ansprüche, **dadurch gekennzeichnet, dass** der Ausleger (3) mindestens teilweise bogenförmig ausgebildet ist, wobei der vom Bogen eingeschlossene Winkel bevorzugt 90° beträgt.

8. Robotisches Operationssystem nach einem der Ansprüche 1 bis 6, **dadurch gekennzeichnet, dass** der Ausleger (3) als von der Tragsäule (2) in einem Winkel von 90° abstehend ausgebildet ist.

9. Robotisches Operationssystem nach einem der vorgenannten Ansprüche, **dadurch gekennzeichnet, dass** der Ausleger (3) in seiner Länge verstellbar ist.
